# EUROPÄISCHE PATENTSCHRIFT

(11) **EP 2 126 582 B1**
(45) Veröffentlichungstag und Bekanntmachung des Hinweises auf die Patenterteilung: **06.07.2011**
(21) Anmeldenummer: 08706848.2
(22) Anmeldetag: 28.01.2008
(51) Int. Cl.: G01N 33/68, G01N 33/564

(54) **VERFAHREN ZUM NACHWEIS VON ANTIKÖRPERN AUS KÖRPERFLÜSSIGKEITEN DURCH EINE IMMUNREAKTION MIT GLYKOPROTEIN 2 (GP2) AUS ZYMOGENEN GRANULA DES PANKREAS ZUR DIFFERENTIALDIAGNOSE VON ENTZÜNDLICHEN DARMERKRANKUNGEN UND CHRONISCHER PANKREATITIS**
METHOD FOR ASSAYING ANTIBODIES IN BODY FLUIDS BY IMMUNE REACTION WITH GLYCOPROTEIN 2 (GP2) FROM ZYMOGENIC GRANULES OF THE PANCREAS FOR THE DIFFERENTIAL DIAGNOSIS OF INFLAMMATORY INTESTINAL DISEASES AND CHRONIC PANCREATITIS
PROCEDE A LA VERIFICATION D'ANTICORPS DANS DES LIQUIDES CORPORELS PAR UNE REACTION IMMUNITAIRE AVEC LES GLYCOPROTEINES 2 (GP2) A PARTIR DE GRANULES ZYMOGENES DU PANCREAS POUR LE DIAGNOSTIC DIFFERENTIEL DE MALADIES INFLAMMATOIRES DE L'INTESTIN ET DE PANCREATITES CHRONIQUES

(30) Priorität: 26.01.2007 EP 07090010; 26.01.2007 DE 102007004909
(43) Veröffentlichungstag der Anmeldung: 02.12.2009
(73) Patentinhaber: GA Generic Assays GmbH, 15827 Dahlewitz (DE)
(72) Erfinder: ROGGENBUCK, Dirk, 15344 Strausberg (DE)
(74) Vertreter: Boeckh, Tobias
(86) Internationale Anmeldenummer: PCT/DE2008/000183
(87) Internationale Veröffentlichungsnummer: WO 2008/089756

(56) Entgegenhaltungen:
- WO-A-01/94409
- WO-A-96/17873
- WO-A-2006/022643
- JP-A- 2004 005 319
- US-A- 6 100 098
- BOSSUYT X: "Serologic Markers in Inflammatory Bowel Disease" CLINICAL CHEMISTRY, Bd. 52, Nr. 2, 2006, Seiten 171-181, XP002491953 in der Anmeldung erwähnt
- PEETERS M ET AL: "Diagnostic value of anti-Saccharomyces cerevisiae and antineutrophil cytoplasmic autoantibodies in inflammatory bowel disease" AMERICAN JOURNAL OF GASTROENTEROLOGY, NEW YORK, NY, US, Bd. 96, Nr. 3, 1. März 2001 (2001-03-01), Seiten 730-734, XP002445952 ISSN: 0002-9270
- FUKUOKA S-I: "Molecular cloning and sequences of cDNAs encoding alpha (large) and beta (small) isoforms of human pancreatic zymogen granule membrane-associated protein GP2" BIOCHIMICA ET BIOPHYSICA ACTA . GENE STRUCTURE AND EXPRESSION, ELSEVIER, AMSTERDAM, NL, Bd. 1491, Nr. 1-3, 25. April 2000 (2000-04-25), Seiten 376-380, XP004275651 ISSN: 0167-4781 -& DATABASE UniProt [Online] XP002440349 Database accession no. P55259
- WONG S M E ET AL: "Sequence of the cDNA encoding human GP-2, the major membrane protein in the secretory granule of the exocrine pancreas" GENE, ELSEVIER, AMSTERDAM, NL, Bd. 171, Nr. 2, 1. Juni 1996 (1996-06-01), Seiten 311-312, XP004042819 ISSN: 0378-1119 & DATABASE UniProt [Online] XP002440349 Database accession no. P55259

## Beschreibung

Die Erfindung betrifft ein Verfahren zum Nachweis von Antikörpern aus Stuhl und/oder Körperflüssigkeiten durch eine Immunreaktion mit GP2 aus zymogenen Granula des Pankreas oder dessen immunreaktive Sequenzen

Das Verfahren kann zur Diagnose oder Therapiekontrolle von Erkrankungen dienen, die mit einer Immunreaktion gegen GP 2 und dessen Analoge Substanzen einhergehen. Gegenstand der Erfindung ist daher die Verwendung von GP2, oder dessen immunreaktive Sequenzen zur Diagnose oder Therapiekontrolle von chronisch entzündlichen Darmerkrankungen, ausgewählt aus der Gruppe umfassend Morbus Crohn (MC) Chronischer Pankreatitis (CP), und Colitis ulcerosa (CU).

Die vorliegende Erfindung stützt sich auf die Erkenntnis, dass GP2 ein Autoantigen immuner Prozesse bei entzündlichen Darmerkrankungen (EDE) bevorzugt bei MC und CP ist und daher ein Epitop der krankheitsassoziierten Antikörper darstellt.

GP2 ist ein Membranglykoprotein der azinösen Zellen des Pankreas mit einem apparenten Molekulargewicht von 78 kD. Weiterhin wurde GP2 in den Bürstensaumzellen des Darms nachgewiesen sowie als Bestandteil von Lysosomen oder als freies, nichtmembrangebundenes Peptid im Pankreassaft. Es stellt mit 30-45% des Gesamtmembranproteins die Hauptkomponente der zymcgenen Granulamembran. Zusammen mit anderen sekretorischen Pankreasproteinen der zymogenen Granula wie Syncollin, Lektin ZG16p, Synaptobrevin 2 und verschiedenen Sulfatmatrixproteoglykanen ist GP2 ein Bestandteil von Lipidflößen (lipid rafts) der Granulamembran, wobei Syncollin mit GP2 interagiert. Diese Komplexe, zu denen noch andere Proteine wie ZG46p gehören können, bilden die submembranöse Matrix.

Über einen Phosphatidylinositolanker ist GP2 an die Membran der zymogener Granula der azinösen Zellen des Pankreas gebunden und kann z.B. durch Phospholipase C von B. cereus abgespalten werden. Zymogene Granula sind die Vorratskammern von Verdauungsenzymen wie zum Beispiel Amylase in den azinösen Zellen des Pankreas. Nach neuronaler oder hormonaler Stimulation der azinösen Zellen werden die Verdauungsenzyme in die Pankreasgänge sekretiert. Neben der Lokalisation von GP2 auf der Membran zymogener Granula wird es auch in deren Matrix, im Golgi Apparat sowie im Lumen der Azini im Pankreassaft gefunden. Weiterhin gibt es Hinweise, dass GP2 ein Bestandteil von Lysosomen und somit an der Endozytose beteiligt ist.

Während der Stimulation der Pankreassekretion wird GP2 zur apikalen Membranoberfläche der azinösen Zellen transportiert, abgespalten und in das Azinuslumen abgegeben. Aufgrund der relativ großen Menge an GP2 im Pankreassaft wird spekuliert, dass es einen weiteren zellulären Pool gibt, von dem GP2 sekretiert werden kann. GP2 wird im Gegensatz zu den Verdauungsenzymen, die durch Proteolyse im Darm aktiviert werden, bereits in der Azinuszelle durch Spaltung verändert. Es wird vermutet, dass die intrazelluläre sequentielle Proteolyse des GP2 dessen Funktion beeinflusst.

Weil die Serumspiegel von GP2 bei akuter und chronischer Pankreatitis erhöht sind, wird die Eignung von GP2 als Marker für die serologische Diagnostik dieser Entitäten diskutiert. In einem Rattenmodell konnte gezeigt werden, dass die Serumkonzentration von GP2 mit dem Schweregrad entzündlicher Darmerkrankungen korreliert.

Für humane Zelllinien konnte dieser Zusammenhang bislang nicht zweifelsfrei erbracht werden, die Spiegel des über die Autoantikörper detektierbaren GP2 weisen eine erhebliche individuelle Variabilität auf.

Trotz dieses Nachteils setzen eine Reihe von Ansätzen zur Entwicklung diagnostischer Verfahren setzen auf die Herstellung und Detektion der Antikörper, die gegen GP2 gerichtet sind, um anhand der Antikörper-Reaktion den Schweregrad entzündlicher Darmerkrankungen zu ermitteln.

Unter den entzündlichen Darmerkrankungen stellen MC und Colitis ulcerosa (CU) die zwei wichtigsten dar. Sie sind durch chronische, rezidivierende, Gewebe zerstörende Entzündungsprozesse im Verdauungssystem gekennzeichnet. Die Ätiologie und Pathogenese von MC wie auch CU sind bisher unklar.

Während bei der CU die Entzündung vor allem in der Mukosa und Submukosa des Kolon und Rektum auftritt, sind beim MC wanddurchgreifende, granulomatöse Entzündungsprozesse des gesamten Gastrointestinaltrakts charakteristisch.

Genetische wie auch Umweltfaktoren scheinen eine entscheidende Rolle bei der Ausbildung von EDE zu besitzen. Der Zusammenhang zwischen Mutationen im NOD2-Gen und Auftreten des MC ist in mehreren Kohorten als gesichert anzusehen. Eine klare Assoziation besteht ebenfalls zum Auftreten des MC im terminalen lieum. Ein Bezug zwischen genetischen Markern und Therapieverlauf konnte bislang für kein Behandlungsverfahren (inklusive der anti-TNF Therapie) etabliert werden.

Die Inzidenz von MC in Europa liegt bei 5,6 pro 100.000 pro Jahr. Die Prävalenz von MC in Deutschland wird mit 1/500 bis 1/800 angegeben.

Erste Krankheitssymptome von MC treten im Mittel relativ früh mit 30 Jahren auf. MC Patienten sind somit in ihrem Berufsleben betroffen, was entsprechende sozioökonomische Effekte nach sich zieht. Ähnlich wie bei der CU ist bei MC Patienten mit Crohn Colitis und langjährigem Verlauf die Karzinom-Inzidenz erhöht.

Das Beschwerdebild umfasst Unterleibsschmerzen, Durchfall, Malabsorbtion, Abszesse, Fisteln, Gallensteinkomplikationen, Nierensteine und deren Komplikationen.

MC Patienten können eine Reihe von extraintestinalen Manifestationen aufweisen, wobei die Pankreatitis mit 3,5% relativ selten bei MC Patienten vorkommt. Eine Hyperamylasämie und Hyperlipasämie ohne Zeichnen- einer akuten Pankreatitis kann jedoch bei 8-17% der Patienten beobachtet werden, was auf eine höhere Rate von silenter Pankreatitis hinweist. Vereinzelt sind Veränderungen im Pankreasgang und Einschränkungen der Pankreasfunktion beschrieben worden. Die Höhe der Hyperamylasämie und Hyperlipasämie korreliert mit der Aktivität von MC . Bei 4,6% der MC Patienten findet man gleichzeitig Veränderungen im Gallen- und Pankreasgang ähnlich wie bei Patienten mit Primär Sklerosierender Cholangitis. Die chronische Pankreatitis bei MC Patienten unterscheidet sich allerdings im Allgemeinen von jener bei CU, welche häufiger eine Beteiligung der Gallengänge, Gewichtsverlust und Pankreasgangstenosen aufweist. Es wird die Existenz einer idiopathischen chronischen Pankreatitis, welche mit MC assoziiert ist, diskutiert. Im Unterschied zur CU assoziierten chronischen Pankreatitis treten bei MC Patienten die intestinalen Symptome häufiger vor dem Erscheinen pankreatischer Befunde auf. Die häufige exokrine Pankreasinsuffizienz bei MC lässt sich leicht auf die ausgeprägte azinöse Degeneration zurückführen, die mit dichten Entzündungsinfiltraten im Parenchym einhergeht.

Als Therapie wird die Gabe von 5-Aminosalizylsäure empfohlen, obwohl verschieden Studien auf erhebliche Nachteile aufmerksam gemacht haben, weil mit diesem Wirkstoff nur begrenzte oder keine Effekte erzielt wurden.. Die Anwendung erscheint jedoch bei Patienten mit leichtem bis mäßig schwerem Schub aufgrund der vorhandenen Daten durchaus gerechtfertigt, wenn man bei Wirkungslosigkeit rechtzeitig einen Therapiewechsel einleitet. Bei schwerem Schub ohne Komplikationen ist die Gabe von Prednisolonäquivalenten in Erwägung zu ziehen. Liegen häufige Schübe (≥ 2/Jahr) vor, kann zusätzlich Azathioprin oder 6-Mercaptopurin gegeben werden.

Die Gesamtkosten eines Patienten mit MC werden in Deutschland auf 20.000 EUR pro Jahr und Fall geschätzt. Die Aufwendungen für MC Patienten einschließlich indirekter Kosten belaufen sich in Deutschland auf geschätzte 2 Milliarden EUR, in den USA werden für beide EDE 2,6 Milliarden US Dollar als sozioökonomische Kosten angegeben.

Anti-TNFalpha Präparate sind bei MC wirksam und induzieren die Remission der chronischen Erkrankung. Nachteilig sind bei diesen Präparate allerdings die Nebenwirkungen, deshalb kommt ihre Verwendung allein als Reservemedikament in Abhängigkeit von der klinischen Situation in Frage. Lediglich MC Patienten mit aktiver Spondylathropathie als extraintestinale Komplikation scheinen von einer anti-TNFalpha Therapie hinsichtlich beider Beschwerdebilder zu profitieren.

Für eine adäquate Therapie und Verlaufskontrolle dieser Patienten ist eine klare Diagnosestellung notwendig. Ein erheblicher Nachteil ist hierbei allerdings, das bislang keine eindeutigen Tests existieren, und deshalb auf eine klinische Diagnostik des MC zurückgegriffen werden muss, die als essentiellen Bestandteil Ileokoloskopische Segmentbiopsien beeinhaltet, welche auch vor selektiven Darmoperationen indiziert sind. Sie ist im Verlauf jedoch nicht regelmäßig bei jeder akuten Symptomatik bzw. vor einer neuen antientzündlichen Therapie erforderlich. Eine obere endoskopische Diagnostik sollte in der Primärdiagnostik bei jedem Patienten erfolgen.

Im Rahmen der Diagnostik bilden aufwendige und vergleichsweise konstenintensive histologische Untersuchungen von Mukosabiopsien einen weiteren wichtigen Baustein. Dafür werden Biopsien vor allem aus makroskopisch auffälligen und unauffälligen Arealen entnommen. Um die Möglichkeiten der histopathologischen Differentialdiagnostik effizient nutzen zu können, müssen allerdings Biopsien aus mindestens fünf verschiedenen anatomischen Segmenten des gesamten Kolon einschließlich des Rektum, aus dem terminalen Ileum und aus dem oberen Magen-Darm-Trakt entnommen werden.

Der transabdominale Ultraschall als bildgebendes Verfahren dient als sensitives Verfahren zum Nachweis entzündlicher Darmwandveränderungen und der Detektion von Abszessen, Fisteln und Stenosen bei MC Patienten. Ein nachweisbarer erhöhter Blutfluss sowohl in den Mesenterialarterien als auch in der Darmwand ist mit dem Vorliegen akuter Entzündung assoziiert. Endorektaler Ultraschall und die Magnetresonanztomographie (MRT) des kleinen Beckens sind als gleichwertig sensitive Verfahren zur Diagnostik und Klassifikation anorektaler Fisteln und Abszesse anerkannt.

Da es keine eindeutigen Tests gibt muß auf eine Reihe von Parametern für die Labordiagnostik des MC erfasst werden. Die Die Bestimmung von C-reaktivem Protein (CRP), Thrombozyten, Hämoglobin (Hb)/Hämatokrit sowie Leukozyten stellt hierbei die Basisdiagnostik dar. Weitere Parameter wie das Differential-Blutbild und das Albumin werden alsErgänzung herangezogen. In der Akutphase des MC sind die oben genannten Parameter wie CRP und die Leukozytenzahl sowie Akutphasen-Proteine bei vielen Patienten erhöht und werden auch für die Verfaufskontrolle empfohlen.

In der Akutphase kommt es zu einem Anstieg der Darmpermeabilität, der alpha1-Antitrypsin-Clearance und der Ausscheidung von Calprotectin im Stuhl.

Die Erhebung klinischer und histologischer Daten erlauben jedoch keine klare Unterscheidung von MC und CU. Dieser Nachteil führt häufig zur Definition einer Colitis indeterminata (CI). Weiterhin können Darminfektionen wie auch funktionelle Erkrankungen ähnliche Symptome entwickeln und die Differentialdiagnose noch erheblich erschweren. Bei 10 bis 15% der Patienten mit EDE ist die Einteilung in CD oder MC aufgrund der Biopsiedaten und einer gewissen Überlappung klinischer Symptome im Bereich des Kolon äußerst schwierig. Patienten mit Cl scheinen häufiger langfristigere Komplikationen und Anastomoseninsuffizienzen nach chirurgischen Eingriffen aufzuweisen als Patienten mit CU. Die Unterscheidung, ob sich Cl Patienten prognostisch in Richtung eines MC oder einer CU entwickeln, hat jedoch einen bedeutenden Einfluss auf Prognose und Krankheitsverlauf sowie die Wahl der medikamentösen Therapie und den Zeitpunkt chirurgischer Eingriffe. Im Krankheitsverlauf lässt sich häufig später nur auf der Basis weiterer klinischer Daten eine Zuordnung vornehmen. Die Unterscheidung von MC und CU ist zum Beispiel die Grundlage für die Entscheidung, ob bei dem Patienten eine ilioanale Pouchanastomose in Betracht gezogen werden kann. Für MC Patienten mit vorwiegendem Befall des Dickdarms (Crohn Colitis) ist dieser chirurgische Eingriff nur in sehr seltenen Fällen angezeigt, während bei der CU diese Methode häufiger indiziert ist. MC Patienten weisen eine deutlich höhere Rate von Anastomoseninsuffizienzen auf, sodass jede chirurgische Intervention gründlich überdacht werden muss.

Im Rahmen der Differentialdiagnose von EDE sind eine Reihe von Antikörpern beschrieben worden, welche mit körpereigenen und Nahrungsmittelantigenen reagieren. Der Nachweis dieser Antikörper ist, dass sie keine pathogenetische Rolle zu spielen scheinen und nicht die Krankheitsaktivität abbilden. Trotzdem wird die serologische Antikörperdiagnostik als entscheidende Hilfe bei der Diagnosestellung genutzt und ist vor allem im Fall der Colitis indeterminata Grundlage für eine Therapieentscheidung.

Autoantikörper gegen Zytoskeletproteine wurden bei mittels Biopsie bestätigten MC Patienten beschrieben (Mayet et al., 1990). Unter anderem wurden Autoantikörper gegen Cytokeratin 18, Aktin, Vimentin, Desmin und Tropomyosin gefunden. Obwohl Cytokeratin 18 Autoantikörper eine Korrelation zur Krankheitsaktivität aufwiesen, haben sie sich in der Routinediagnostik von EDE wahrscheinlich aufgrund der geringen Spezifität nicht durchgesetzt.

Für MC Patienten sind Autoantikörper gegen Gewebe von exokrinem Pankreas (PAK) und Antikörper gegen Mannan von Saccharomyces cerevisiae (ASCA) als pathognomonisch identifiziert worden (Stöcker et al., 1987; Main et al., 1988). Autoantikörper gegen humane neutrophile Granulozyten (ANCA) und Becherzellen (BAK) werden bevorzugt bei CU Patienten gefunden.

Die Bestimmung von PAK, ANCA und ASCA wird für die Diagnosestellung bei Cl als hilfreich eingeschätzt .

EDE spezifische Autoantikörper gegen Pankreasgewebe, Becherzellen und humane neutrophile Granulozyten werden heute jedoch noch mit Hilfe der Immunfluoreszenztechnik (IFT) aufgrund der nicht bekannten, für die Immunreaktion verantwortlichen Autoantigene bestimmt. So wurden mittels dieser Technik bei 27-39% von MC Patienten Autoantikörper gegen Pankreasantigene gefunden. Über die Hälfte der MC Patienten (68%) mit extraintestinalen Komplikationen können PAK aufweisen. In der Praxis erweist sich diese Technik allerdings als nachteilig, da sie nicht automatisierbar und daher kosten- und zeitaufwendig ist.

Für den MC ebenfalls spezifische ASCA werden dagegen verstärkt im Enzymimmunoassay (EIA) detektiert, da diese Methode weniger subjektiv in der Auswertung und automatisierbar ist.

Heute werden die einzelnen Antikörperbestimmungen für die serologische Diagnostik des MC als zu insensitiv angesehen. Die Kombination verschiedener Antikörperspezifitäten kann die diagnostische Sensitivität bzw. Spezifität für die Differentialdiagnose von EDE außerordentlich verbessern und für Cl eine Prognose zulassen.

Für die Kombination von Parametern ist eine gemeinsame Technologieplattform wie die des EIA äußerst vorteilhaft. Das setzt allerdings die Kenntnis des Autoantigens der PAK voraus, welches von den PAK in den Pankreasgewebeschnitten unterschiedlicher Spezies spezifisch erkannt wird.

Es hat verschiedene Ansätze in der Vergangenheit zur Identifizierung der Autoantigene bei MC gegeben, wobei Pankreasantigene aufgrund der bereits genannten relativen hohen Sensitivität der PAK im Mittelpunkt des Interesses standen. PAK wurden mittels Gewebeschnitten unterschiedlicher Spezies (human, Ratte, Affe) in der IFT nachgewiesen. Das lässt hinsichtlich der Phylogenese auf konservierte Epitope schließen, die von den PAK erkannt werden. Fricke et al. beschrieben einen Proteinkomplex bestehend aus mehreren mit PAK reagierenden Untereinheiten mit einem Molekulargewicht (MW) größer als 800 kDa (Fricke et al., 1999). Die Autoren waren jedoch nicht in der Lage das entsprechende Protein noch die im Immunoblot mit PAK reaktiven Untereinheiten mit den MW 16, 18, 19, 24, 27, 29, 31 und 34 kDa zu sequenzieren und damit zu identifizieren. Es wurde vermutet, dass das von PAK erkannte Protein ein großer Proteinkomplex mit mehreren Untergruppen zu sein scheint. Aufgrund von Inhibitionsexperimenten mit verschiedenen Glykoproteinen wurde eine Reaktivität der PAK mit Kohlenhydratketten der putativen Autoantigene ausgeschlossen.

Seibold et al. beschrieben die Reaktivität von PAK gegen ein aus Pankreassaft gereinigtes makromolekulares Antigen mit einem MW von größer als 10⁶ Da (1000 kDa), welches nach Trypsinbehandlung seine PAK Reaktivität verlor. Im ELISA mit verschiedenen Pankreasproteinen wie Amylase, Lipase, Phospholipase A und C, Enterokinase, Carboxypeptidase A und B, Chymotrypsin A und B, Chymotrypsinogen, Elastase, Trypsin, Trypsin Inhibitor, Lactoferrin und Kallekrein konnte keine Reaktivität mit PAK festgestellt werden.

Obwohl der Spiegel von GP2 und der Krankheitsgrad von EDE nach einigen Autoren miteinander korrelieren, ist der physiologische Zusammenhang unbekannt. In einem Knock-out Mausmodell konnte gezeigt werden, dass die Abwesenheit von GP2 weder für die Sekretion des exokrinen Pankreas noch für die Bildung von zymogenen Granula essentiell ist. Unklar ist weiterhin auch die physiologische Funktion der beiden bekannten Isoformen von GP2 (Fukuoka, 2000). Neben einer kurzen Isoform mit einer Länge von 380 Aminosäuren, β-GP2, exisitiert eine 530 Aminosäuren Lange α-GP2. Beide Isoformen des Peptids sind u.a. im Gewebe des menschlichen Pankreas nachweisbar, wobei die kleine β-GP2 Isoform in erheblich höheren Titern auftritt als die große α-GP2. Auch ein Nachweis der Transkripte beider Formen im Gewebe des Pankreas zeigt eine stärkere Expression von β-GP2 als von α-GP2.

Die Rolle der veschiedenen Isoformen ist ungeklärt. Peptide, deren Sequenzen hohe Ähnlichkeiten mit der großen Isoform α-GP2 aufweisen sollen verantwortlich sein für die Bildung von Pankreas-Tumoren. Antikörper gegen GP2 als Analyt und Marker sollen für die Diagnose von Pankreaskrebs verwendet werden und das Peptid und seine Nucleinsäuresequenz für die Immuntherapie von Krebserkrankungen des Pankreas (WO 01/94409). Antikörper gegen die kleine Isoform β-GP2 finden als Marker für Pankreatitis Verwendung (WO 96/17873). Die Zunahme der β-GP2 Konzentration soll die Erkrankung nachweisen.

Ausdrücklich wurde auf die Notwendigkeit der ausstehenden Identifizierung des (der) Autoantigens(e) des Pankreas hingewiesen, um den Stellenwert von autoimmunen Prozessen in der Pathogenese des MC aufzuklären und die Diskriminierung von unklaren EDE Fällen durch eine entsprechende Labordiagnostik zu unterstützen.

Im Stand der Technik sind Versuche bekannt, bei denen mittels randomisierter Phagendisplay Bibliotheken Peptide gewonnen werden konnten, die für die Bestimmung von MC spezifischen Antikörpern eingesetzt wurden. Es wurden 4 verschiedene Nonamere bestimmt, die bei Verwendung in einem EIA 56,5% der Seren von MC Patienten positiv detektierten. Kontrollgruppen (CU, Duodenale Geschwüre, Gesunde) reagierten nicht oder nur in 6% der Fälle. Aufgrund der bekannten Peptidsequenzen der Nanomere konnte jedoch kein Rückschluss auf das oder die nativen Autoantigen(e) des MC gezogen werden.

Bislang ist es jedoch nicht gelungen, das (die) entsprechende(n) Pankreasantigen(e) zu identifizieren (Bossuyt, 2006). Somit existiert bis heute kein nicht-invasiver, spezifischer, quantitativer, schnell, leicht und kostengünstig durchzuführender Nachweis zur Diagnose von Morbus Crohn und chronischer Pankreatitis sowie deren Diskriminierung gegenüber Colitis ulcerosa.

Dieses Problem wird durch die Erfindung gelöst. Basierend auf der überraschenden Charakterisierung von GP2 als Autoantigen für MC und die assoziierte chronische Pankreatitis wurde ein Verfahren unter Verwendung von GP2 gemäß der Ansprüche zur Diagnose oder Therapiekontrolle von EDE entwickelt, wobei sich vorteilhafte Ausführungsformen der Erfindung aus den Unteransprüchen ergeben.

Die Erfindung betrifft demgemäß ein Verfahren zum Nachweis von Antikörpern aus Stuhl und/oder Körperflüssigkeiten insbesondere Blut und/oder Serum für die Diagnose und/oder Therapiekontrolle von entzündlichen Darmerkrankungen, ausgewählt aus der Gruppe umfassend Morbus Crohn, chronische Pankreatites und/oder Colitis ulcerosa, durch eine Immunreaktion mit GP2 gemäß SEQ ID Nr. 1, oder einem Molekül aufweisend eine Aminosäuresequenz, die eine Homologie zu SEQ ID Nr. 1 aufweist, wobei die Homologie mindestens 80%, vorzugsweise 90% beträgt, wobei die lmmunreaktion eine Bindung eines Antikörpers an ein Antigen umfasst und GP2 gemäß SEQ ID Nr. 1 das Antigen ist. SEQ ID Nr. 1 hat die folgende Aminosäuresequenz:

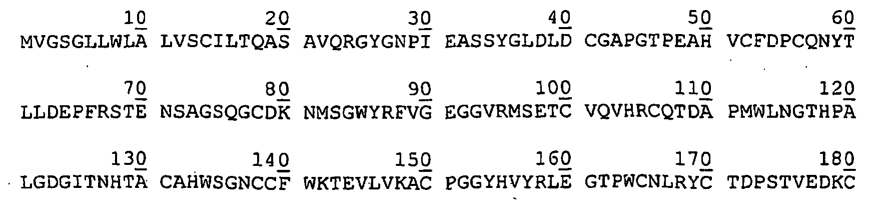

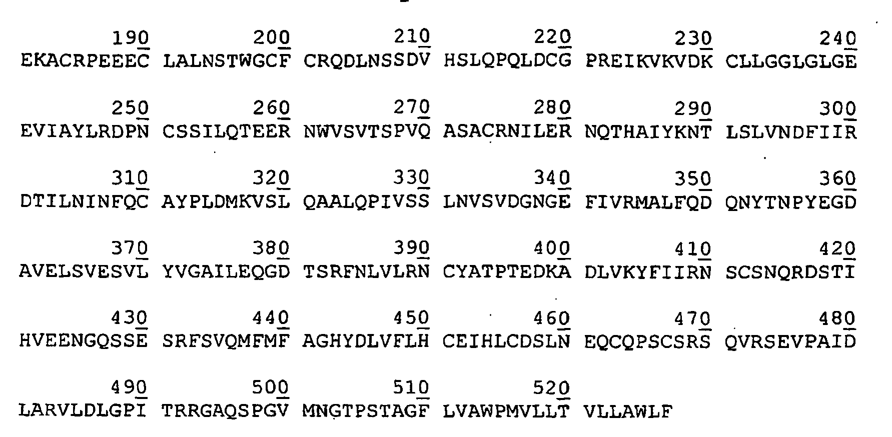

Als Körperflüssigkeiten werden erfindungsgemäß außerdem definiert menschliches Blut, Serum, Urin, reine pankreatische Säfte oder duodenale Säfte.

Die Bestimmung von Autoantikörpern gegen GP2 oder deren Verwendung in einem ELISA als Festphasenantigen für die serologische Diagnostik von MC oder der chronischen Pankreatitis ist im Stand der Technik weder in Betracht gezogen noch erwähnt worden.

Gegenstand der Erfindung ist die überraschende Lehre, dass GP2, insbesondere gemäß der Sequenz SEQ ID Nr. 1 zum Nachweis der entzündlichen Darmerkrankungen. Morbus Crohn, chronische Pankreatitis und Colitis ulcerosa verwendet werden können.

Ein weiterer Aspekt der Erfindung betrifft ein Verfahren, bei dem humane 1gA-, IgM und/oder 1gG-Antikörper Autoimmunerkrankungen nachgewiesen werden.

In einer bevorzugten Ausführungsform des erfindungsgemäßen Verfahrens ist das GP2 humanen, tierischen, rekombinanten oder synthetischen Ursprungs. Da GP2 ein hochkonserviertes Peptid darstellt, kann der Nachweis vorteilhafterweise GP2 jedweden Ursprungs verwenden, solange die Sequenz funktionsanalog ist zur erfindungsgemäßen Sequenz. Die hohe Bindungsaffinität zwischen GP2 als Antigen und den Autoantikörpern bleibt bestehen.

In einer weiteren bevorzugten Ausführungsform des erfindunsgemäßen Verfahrens erfolgt der Nachweis der Autoantikörper in einem Immunoassay, vorzugsweise unter direkter oder indirekter Kopplung eines Reaktionspartners mit einer Markierungssubstanz. Dies erlaubt die flexible Anpassung des Verfahrens an die Möglichkeiten und Erfordernisse verschiedener Labore und deren labordiagnostische Ausstattung.

In einer vorteilhaften Ausführungsform erfolgt der Nachweis der EDE spezifischen Antikörper in einem Immunoassay, wobei die Antikörper gelöst in einer flüssigen Phase vorliegen, vorzugsweise verdünnt in einer dem Fachmann bekannten üblichen Pufferlösung oder in einer unverdünnten Körperflüssigkeit. Weiterhin kann der Nachweis erfindungsgemäß auch aus Proben des Stuhls erfolgen.

In einer weiteren vorteilhaften Ausführungsform dient der Immunoassay dem Nachweis von Antikörpern und sieht hierzu die Bindung des Antigens GP2 gemäß der SEQ ID Nr. 1 an eine feste Phase vor. Nach Zugabe der Probenlösung bindet der darin enthaltene Antikörper eines Patienten an das Antigen GP2. Der durch GP2 gebundene Antikörper, der z.B. aus dem Serum oder Stuhl eines Patienten stammt, wird anschließend durch ein markiertes Reagens nachgewiesen und gegebenenfalls quantifiziert. Dieses Verfahren, bei dem das Antigen an die feste Phase gebunden ist, ist dem Fachmann als Direct Assay bekannt.

Erfindungsgemäß erfolgt die Detektion der Antikörper in diesem Verfahren also durch den Einsatz markierter Reagenzien nach bekannter ELISA (Enzyme-linked Immunosorbent Assay) Technologie. Erfindungsgemäße Markierungen umfassen daher Enzyme, die eine chemische Reaktion katalysieren, die optisch bestimmt werden kann, insbesondere durch chromogene Substrate, Chemilumineszenz-Verfahren oder Fluoreszenz-Farbstoffen.

In einer weiteren bevorzugten Ausführungsform erfolgt die Detektion derAutoantikörper durch Markierung mit schwach radioaktiven Substanzen in Radioimmunassays (RIA), wobei die auftretende Radioaktiviät gemessen wird.

In einer weiteren bevorzugten Ausführungsform der Erfindung werden lösliche oder festphasengebundene GP2-Moleküle zur Bindung von Antikörpern verwendet. In einem zweiten Reaktionschritt werden anti-human-Immunglobiline, vorzugsweise ausgewählt aus der Gruppe umfassend anti-human IgA-, anti-human IgM und/oder anti-human IgG-Antikörper, wobei es sich bei den anti-human Immunoglobulinen um detektierbar markierte Konjugate von zwei Komponenten handelt, die mit allen üblichen Markierungsenzymen, insbesondere chromogene und/oder chemilumineszenz Substrate, konjugiert werden können, vorzugsweise mit: - Meerrettichperoxidase - alkalischer Phosphatase. Der Vorteil dieser Ausführungsform ist die Verwendung von üblicherweise in Laborbetrieben vorhandener ELISA-Technologie, so dass der erfindungsgemäße Nachweis kostengünstig etabliert werden kann.

In einer weiteren bevorzugten Ausführungsform der Erfindung reagiert der an GP2 gebundene Antikörper mit anti-human-Immunglobilinen, vorzugsweise ausgewählt aus der Gruppe umfassend anti-human IgA-, anti-human IgM und/oder anti-human IgG-Antikörper, die detektierbar an Fluorescein-Isothiocynat (FITC) gekoppelt sind. Wie der o.g. ELISA-Test, so stellt auch die FITC-Technologie ein System dar, dass vielerorts etabliert ist und daher eine reibungslose und kostengünstige Etablierung des erfindungsgemäßen Nachweises in die Laborroutine erlaubt.

In einer bevorzugten Ausführungsform des vorgenannten erfindungsgemäßen Verfahrens erkennt GP2 gemäß SEQ ID Nr. 1 gegen Darm-Gewebe gerichtete Autoantikörper. Diese Ausführungsform hat den vorteilhaften Effekt, dass die Diagnose von EDE für den Patienten ohne vielfach belastende Eingriffe einfach aus Körperflüssigkeiten oder Stuhl erfolgen kann.

In einer weiteren bevorzugten Ausführungsform der Erfindung ist das GP2 gemäß SEQ Nr. 1 an eine Festphase gebunden. Die Bindung des GP2 gemäß SEQ Nr. 1 an die Festphase kann über einen Spacer erfolgen. Als Spacer können alle chemischen Verbindungen eingesetzt werden, die für die Funktion des Spacers die geeigneten strukturellen und funktionellen Voraussetzungen aufweisen, solange sie nicht das Bindeverhalten derart modifizieren, dass eine Bindung des Autoantikörpers mit GP2 gemäß SEQ Nr. 1 nachteilhafterweise beeinträchtigt wird.

Die erfindungsgemäßen Verfahren und Verwendungen ermöglichen die Diagnose oder Therapiekontrolle von Morbus Crohn, weil es mit dem Antigen GP2 bevorzugt gemäß SEQ Nr. 1 überraschenderweise möglich ist, Autoantikörper aus Stuhl und/oder Körperflüssigkeiten, insbesondere Blut und/oder Serum durch eine Immunreaktion mit GP2, dessen immunreaktiven Sequenzen oder Analoga nachzuweisen, wobei diese Immunreaktion nicht mit einem Gewebeschnitt eines tierischen oder menschlichen Gewebes durchgeführt wird.

In einer bevorzugten Ausführungsvariante des beschriebenen Nachweisverfahrens wird der Autoantikörper vorzugsweise unter Verwendung direkter oder indirekter Kopplung eines Reaktionspartners mit einer Markierungssubstanz detektiert.

Erfindungsgemäß ist weiterhin die Durchführung des beschriebenen Nachweisverfahrens an einer festen Phase, wobei die überraschend stabile Bindung des Antigens GP2 an die feste Phase vorteilhafter Weise die Lagerfähigkeit des Peptids erhöht.

Gegenstand der Erfindung ist weiterhin die Verwendung des Moleküls GP2 gemäß der Sequenz SEQ ID Nr. 1 zur Herstellung eines Medikaments zur Anwendung in der Diagnose und/oder Therapie kontrolle von entzürdlichen Darmerkrankungen. In der Praxis hat diese erfindungsgemäße Verwendung den vorteilhaften Effekt, dass auf aufwendigere und unsichere Verfahren, die sich etwa auf Biopsien, Labordiagnostik und klinische Eingriffe stützen, vielfach verzichtet werden kann oder die Maßnahmen aufgrund des bestehenden Nachweises deutlich optimiert vorgenommen werden können. GP2 stellt hierbei ein ausreichend stabiles und Resorbierbases Peptid dar, so dass es Patienten als Medikament sogar in oraler Form Vesabreicht werden kann.

In der Erfindung ist die entzündliche Darmerkrankung Morbus Crohn, chronische Pankreatitis und/oder Colitis ulcerosa. Diese Krankheiten konnten bisland nur sehr schlecht oder mit sehr großem Aufwand nachgewiesen bzw. voneinander unterschieden werden. Durch diese bevorzugte Ausührungsform ist es nun möglich, Morbus Crohn und chronische Pankreatitis einfach nachzuweisen und sogar von Colitis ulcerosa auf dem Wege der Differentialdiagnostik zu unterscheiden.

Der Morbus Crohn gehört zur Gruppe der chronisch-entzündlichen Darmerkrankungen. Es handelt sich um eine vermutlich autoaggressive, chronischgranulomatöse Entzündung, die im gesamten Magen-Darm-Trakt von der Mundhöhle bis zum After auftreten kann. Bevorzugt befallen sind der untere Dünndarm *(terminales Ileum,* Befall in zirka 40 %) und Grimmdarm, seltener. Speiseröhre *(Ösophagus)* und Mund. Charakterisierend für Morbus Crohn ist der diskontinuierliche, segmentale Befall (sog. "*skip lesions")* der Darmschleimhaut, d. h. es können gleichzeitig mehrere Darmabschnitte erkrankt sein, die durch gesunde Abschnitte voneinander getrennt sind. Andere Bezeichnungen für die Krankheit sind Enteritis regionalis Crohn, lleitis terminalis, Enterocolitis regionalis und sklerosierende chronische Enteritis, bzw. die Abkürzungen MC, CD *(Crohn's Disease)* und übergreifend als IBD *(inflammatory Bowel Disease).* Morbus Crohn im Sinne der Erfindung ist demgemäß jeder Zustand, der sich makroskopisch durch folgende Veränderungen charakterisieren lässt:
- Gartenschlauchphänomen: Durch Fibrosierung verursachte Segmentstenosen
- Pflastersteinphänomen: Entzündete Schleimhaut wechseln sich mit tiefen Ulzerationen ab, wodurch ein pflastersteinartiges Aussehen entsteht.
- Entzündlicher Konglomerattumor: Verschiedene Darmabschnitte verkleben miteinander.

Histologisch (feingeweblich) erkennt man vor allem eine Häufung von Lymphozyten, (eosinophilen) Granulozyten und Histiozyten in der Biopsie des entzündeten Darmgewebes. Angrenzende Lymphknoten sind meist vergrößert. Häufig bilden sich Granulome, die sich in zwei Typen unterscheiden lassen: *Epitheloidzellgranulome* und *Mikrogranulome* (kleiner und ohne zentrale Nekrose).

Morbus Crohn im Sinne der Erfindung lässt sich aber auch diagnostisch charakterisieren. In diesem Falle ist Morbus Crohn im Sinne der Erfindung ein Zustand, bei dem mindestens eines der folgenden Merkmale detektierbar ist:
- Appendizitis: meist ein sich rasch entwickelnder Schmerz im rechten Unterbauch. Häufig eine Temperaturdifferenz > 1°C zwischen rektaler und axillärer Messung.
- Divertikulitis: tastbare Resistenzen bei meist linksseitigem Unterbauchschmerz.
- Yersiniose: Erregernachweis aus dem Stuhl oder aus dem Biopsiematerial, Anstieg des Antikörpertiters.
- Darmtuberkulose: In Mitteleuropa mittlerweile sehr selten. Die Darmtuberkulose geht häufig mit Beteiligung der Lunge einher. Es finden sich "verkäsende" epitheloidzellige Granulome im Biopsiematerial.
- jede andere invasive infektiöse Colitis (Salmonellenenteritis, pseudomembranöse Colitis etc.)

Pankreatitis ist im Sinne der Erfindung eine Entzündung der Bauchspeicheldrüse *(Pankreas),* die akut oder chronisch verlaufen kann.

In den meisten Fällen entsteht die Pankreatitis durch eine Aktivierung von Pankreas-Enzymen innerhalb des Organs. Da es die Aufgabe dieser Enzyme ist, Eiweiße und Fette zu verdauen, beginnt eine Selbstverdauung des Organs. Diese Selbstverdauung führt zur Entzündung der Bauchspeicheldrüse. In schweren Fällen können Blutungen, ernste Gewebeschäden, Infektionen und Zysten entstehen. Eine entzündete Drüse kann dazu führen, dass Enzyme in den Blutstrom eintreten und so die Lungen, das Herz und die Nieren erreichen, wo weitere Schäden auftreten können. Akute Pankreatitis entsteht, wenn sich die Bauchspeicheldrüse plötzlich entzündet, sich dann aber wieder erholt. Einige Patienten leiden mehrmals an akuter Pankreatitis, können sich aber jedes Mal vollständig erholen. Eine akute Pankreatitis tritt plötzlich auf und kann eine ernste, lebensbedrohliche Krankheit sein, die zahlreiche Komplikationen hervorruft, normalerweise erholen sich Patienten aber von einer akuten Pankreatitis. Die Inzidenz beträgt etwa fünf bis zehn Neuerkrankungen pro 100.000 Einwohner pro Jahr.

Sie kommt in zwei Verlaufsformen vor:
*1. ödematöse Pankreatitis:* blander Verlauf mit Schwellung des Organs und geringen Nekrosen im Fettgewebe der Umgebung
*2. hämorrhagisch - nekrotisierende Pankreatitis:* ausgedehnte Nekrosen und Blutungen im Pankreas und in die Umgebung; durch ihr fulminates Krankheitsbild oft auch als *Pankreasapoplexie* bezeichnet

Die morphologische Beurteilung des Pankreas, insbesondere die Differenzierungzwischen ödematöser und nekrotisierender Pankreatitis gelingt am besten mit der kontrastmittelverstärkten Computertomographie. Zur Einteilung des Schweregrades hat sich der Balthazar-Score bewährt (0 bis 10 Punkte).

Die *akute* Pankreatitis kann mehrere Ursachen haben. Am häufigsten sind Gallensteine, die sich in der Mündung des Gallengangs in den Zwölffingerdarm, die gleichzeitig auch die Mündung des Bauchspeicheldrüsengangs ist, vorübergehend oder länger festklemmen. (ca. 45% der akuten Pankreatitiden). Eine ebenfalls sehr gängige Ursache ist chronischer Alkoholmissbrauch (ca. 35%). Bei etwa 15% der Betroffenen lässt sich kein konkreter Auslöser feststellen, in diesen Fällen spricht man von idiopathischer Genese. Daneben kommen auch seltenere Ursachen vor wie:
- Als Nebenwirkung von Medikamenten (z. B. Asparaginase, Azathioprin, Furosemid, Glukokortikoide, Antibiotika (Tetrazykline, Sulfamethoxazol, Trimethoprim), Antikonvulsiva (Valproat, Carbamazepin), Propofol und andere
- Infektionen z. B. Mumps, Coxsackie-Virus, Hepatitis, HIV, Zytomegalie-Virus
- Erhöhter Blutkalziumwert, z. B. bei Nebenschilddrüsenüberfunktion
- Stark erhöhte Blutfette (Triglyzeride)
- iatrogen nach ERCP
- genetisch: Zystische Fibrose

Eine akute Pankreatitis macht sich anfangs durch Schmerzen im (linken bis gesamten) Oberbauch (Epigastrium), die in den Brustkorb ausstrahlen, bemerkbar, die nach einigen Tagen verschwinden. Die Schmerzen sind oft sehr heftig und zum Teil auch anhaltend. Die Schmerzen können plötzlich und intensiv sein, oder als leichte Schmerzen beginnen und nach der Einnahme von Nahrung (durch die Stimulietung des Pankreas bei der Bildung von Pankreasenzymen zwecks Verdauung der Speise) schlimmer werden. Der Bauch kann geschwollen und sehr empfindlich sein. Charakteristisch bei der körperlichen Untersuchung sind ein druckschmerzhaftes Abdomen und ein sog. *Gummibauch,* der durch Meteorismus und (mäßige) Abwehrspannung bedingt ist. Ebenfalls kann es zu Schmerzen im unteren Bereich der Brustwirbelsäule kommen. Dieser Schmerz ist zunächst ähnlich einem leichten Hexenschuss, entwickelt sich aber in der Folge mehr zu einem "Durchstochenwerden", vom Rücken her hin zum Bereich des Pankreaskopfes auf der Bauchseite.

Patienten mit einer akuten Pankreatitis sehen gewöhnlich sehr krank aus und fühlen sich auch so. Andere Symptome sind zum Beispiel Übelkeit, Erbrechen, Obstipation, Fieber, erhöhter Puls. In schweren Fällen treten Gelbsucht (lkterus) (bei Verlegung der Gallenwege), Bauchwassersucht (Aszites) (bei Verlegung des Pfortadersystems), Pleuraergüsse sowie Schock- und Sepsiszeichen hinzu.

Labordiagnostisch kann eine erhöhte Leukozytenzahl (Leukozytose) sowie ein Anstieg der Konzentration von Pankreasenzymen (z. B. Trypsin, Amylasen, Lipase) nachgewiesen werden. Auch die Calcium-, Magnesium-, Natrium-, Kalium-, Bikarbonat-, Zucker- oder Fettwerte im Blut können erhöht sein.

Ungefähr 20% der akuten Pankreatitis-Fälle sind ernst. Der Patient kann dehydrieren und einen niedrigen Blutdruck entwickeln. Manchmal kommt es zu Herz-, Lungen- oder Nierenversagen. In den schlimmsten Fällen führt eine akute Pankreatitis zu Blutungen, Schock und manchmal zum Tod.

Nach der aktuellen Atlanta-Klassifikation wird die milde von der schweren akuten Pankreatitis unterschieden.

Eine veraltete Einteilung unterteilte eine ödematöse Form (Frühstadium), eine hämorrhagische Form mit lokalen oder generalisierten Blutungen und eine akute nekrotisierende Form.

Eine chronische Pankreatitis hat viele Ursachen, aber 70 bis 80% sind auf chronischen Alkoholmissbrauch zurückzuführen. Sie tritt häufiger bei Männern als bei Frauen auf und entwickelt sich häufig zwischen dem 30. und dem 40. Lebensjahr. Eine chronische Pankreatitis kann sich auch aus einer akuten Entzündung entwickeln, wenn die Ursache nicht beseitigt wird oder der Ausführungsgang beschädigt ist.

Einige chronische Pankreatitiden sind erblich bedingt. Diese beruhen auf einer Abnormalität der vom Pankreas gebildeten Enzyme, welche das Gewebe schädigen. Andere Formen der Erkrankung haben ihre Ursachen in äußeren Faktoren wie z. B. dem Tabakrauchen.

In den frühen Stadien einer Pankreatitis kann der Arzt häufig nicht entscheiden, ob es sich um eine akute oder eine chronische Form handelt. Die Symptome können die gleichen sein.

Eine chronische Pankreatitis verursacht häufig chronische Schmerzen. In einigen Fällen chronischer Pankreatitis lässt der Schmerz nach, wenn die Krankheit fortschreitet. Sie führt auch zu einer Unterfunktion der Bauchspeicheldrüsen-Aktivität, was zu Gewichtsverlust und Verdauungsstörungen führt. Die ungenügende Verdauung und Resorption führt zur Abgabe von Fett und Eiweiß über den Stuhl. Wenn die endokrinen Zellen (Langerhans-Inseln) im Pankreas geschädigt sind, kann sich ein Diabetes entwickeln.

Eine Diagnose der chronischen Pankreatitis ist schwierig, jedoch stehen einige hochentwickelte medizinische Techniken zu Verfügung. Pankreas-Funktions-Bluttests können helfen, zu entscheiden, ob die Bauchspeicheldrüse noch in der Lage ist, genug Verdauungsenzyme herzustellen. Sie haben sich in der Praxis allerdings kaum durchgesetzt.

Abnormalitäten des Pankreas können auch durch Sonografie, ERCP und Computertomographie erkannt werden.

In fortgeschritteneren Stadien einer chronischen Pankreatitis, wenn Diabetes und fehlerhafte Resorption auftreten, kann der Arzt auch Blut, Urin und Stuhltests durchführen, um eine Diagnose zu stellen.

Eine chronische Pankreatitis wird durch das Verschreiben von Schmerzmitteln und eine Nahrungsumstellung behandelt. Patienten können den Verlust von Fett und Eiweiß reduzieren, indem sie Medikamente einnehmen, die Pankreas-Enzyme enthalten. Dies wird eine verbesserte Ernährung und eine Gewichtszunahme zur Folge haben. Manchmal werden Insulin oder andere Medikamente verschrieben, um den Blutzuckerspiegel zu kontrollieren.

In einigen Fällen von chronischer Pankreatitis wird ein chirurgischer Eingriff vorgenommen, um die Schmerzen zu lindern, indem ein vergrößerter und gestauter Pankreasgang entlastet wird.

In einer weiteren bevorzugten Ausführungsform der Erfindung ist die Aminosäuresequenz des Peptids GP2 mindestens zu 80%, bevorzugt 90% homolog zu der Sequenz gemäß SEQ ID Nr. 1. D. h., die Erfindung betrifft sämtliche Peptide, die 80%, bevorzugt 90% Homologie zu der Sequenz gemäß SEQ ID Nr. 1 aufweisen. Selbstverständlich können diese Homologen durch Deletion, Addition, Substitution, Translokation, Inversion und/oder Insertion der sequenz gemäß SEQ ID NO.1 modifiziert sein. Diese Modifikation betrifft homologe Peptide, die funktionsanalog sind. Funktionsanalog sind die Peptide im Sinne der Erfindung, wenn sie mit Autoantikörpern, die mit den o. g. Krankheiten assoziiert sind, spezifisch interagieren.

Erfindungsgegenstand sind demgemäß das offenbarte Peptid sowie die Homologen, die funktionsanalog eingesetzt werden können. Dem Fachmann ist in Bezug auf Homologe/Funktionsanaloge bekannt, dass er Änderungen durch Additionen, Deletionen oder Substitutionen vornehmen kann, ohne dass das Polypeptid im wesentlichen verändert wird. Nicht wesentlich verändert ist die modifizierte Aminosäuresequenz, wenn sie die gleiche Funktion erfüllt, wie die Sequenz gemäß SEQ ID Nr. 1 und zwar im wesentlichen auf dieselbe Art und Weise, wobei sie hierbei zum selben Ergebnis führt.

Funktionsanaloge, Peptide können beispielsweise SEQ ID Nr. 2 
MPHLMERMVGSGLLWLALVSCILTQASAVQRGYGNPIEASSYGLDLDCGAPGTPEAHVCF
DPCQNYTLLDEPFRSTENSAGSQGCDKNMSGWYRFVGEGGVRMSETCVQVHRCQTDAP
MWLNGTHPALGDGITNHTACAHWSGNCCFWKTEVLVKACPGGYHVYRLEGTPWCNLRYC
TVPRDPSTVEDKCEKACRPEEECLALNSTWGCFCRQDLNSSDVHSLQPQLDCGPREIKVK
 
VDKCLLGGLGLGEEVIAYLRDPNCSSILQTEERNWVSVTSPVQASACRNILERNQTHAIYKN
TLSLVNDFIIRDTILNINFQCAYPLDMKVSLQAALQPIVSSLNVSVDGNGEFIVRMALFQDQNY
TNPYEGDAVELSVESVLYVGAILEQGDTSRFNLVLRNCYATPTEDKADLVKYFIIRNSCSNQ
RDSTIHVEENGQSSESRFSVQMFMFAGHYDLVFLHCEIHLCDSLNEQCQPSCSRSQVRSE
VPAIDLARVLDLGPITRRGAQSPGVMNGTPSTAGFLVAWPMVLLTVLLAWLF^
 oder SEQ ID Nr. 3 
MPHLMERMVGSGLLWLALVSCILTQASAVQRGYGNPIEASSYGLDLDCGAPGTPEAHVCF
DPCQNYTLLDEPFRSTENSAGSQGCDKNMSGWYRFVGEGGVRMSETCVQVHRCQTDAP
MWLNGTHPALGDGITNHTACAHWSGNCCFWKTEVLVKACPGGYHVYRLEGTPWCNLRYC
TDPSTVEDKCEKACRPEEECLALNSTWGCFCRQDLNSSDVHSLQPQLDCGPREIKVKVDK
CLLGGLGLGEEVIAYLRDPNCSSILQTEERNWVSVTSPVQASACRNILERNQTHAIYKNTLSL
VNDFIIRDTILNINFQCAYPLDMKVSLQAALQPIVSSLNVSVDGNGEFIVRMALFQDQNYTNP
YEGDAVELSVESVLYVGAILEQGDTSRFNLVLRNCYATPTEDKADLVKYFIIRNSCSNQRDS
TIHVEENGQSSESRFSVQMFMFAGHYDLVFLHCEIHLCDSLNEQCQPSCSRSQVRSEVPAI
DLARVLDLGPITRRGAQSPGVMNGTPSTAGFLVAWPMVLLTVLLAWLF
 oder SEQ ID Nr. 4 
MPHLMERMVGSGLLWLALVSCILTQASAVQRVPRDPSTVEDKCEKACRPEEECLALNSTW
GCFCRQDLNSSDVHSLQPQLDCGPREIKVKVDKCLLGGLGLGEEVIAYLRDPNCSSILQTEE
RNWVSVTSPVQASACRNILERNQTHAIYKNTLSLVNDFIIRDTILNINFQCAYPLDMKVSLQA
ALQPIVSSLNVSVDGNGEFIVRMALFQDQNYTNPYEGDAVELSVESVLYVGAILEQGDTSRF
NLVLRNCYATPTEDKADLVKYFIIRNSCSNQRDSTIHVEENGQSSESRFSVQMFMFAGHYD
LVFLHCEIHLCDSLNEQCQPSCSRSQVRSEVPAIDLARVLDLGPITRRGAQSPGVMNGTPS
TAGFLVAWPMVLLTVLLAWLF
 oder aber SEQ ID Nr. 5 
MPHLMERMVGSGLLWLALVSCILTQASAVQRDPSTVEDKCEKACRPEEECLALNSTWGCF
CRQDLNSSDVHSLQPQLDCGPREIKVKVDKCLLGGLGLGEEVIAYLRDPNCSSILQTEERN
WVSVTSPVQASACRNILERNQTHAIYKNTLSLVNDFIIRDTILNINFQCAYPLDMKVSLQAALQ
PIVSSLNVSVDGNGEFIVRMALFQDQNYTNPYEGDAVELSVESVLYVGAILEQGDTSRFNLV
LRNCYATPTEDKADLVKYFIIRNSCSNQRDSTIHVEENGQSSESRFSVQMFMFAGHYDLVFL
HCEIHLCDSLNEQCQPSCSRSQVRSEVPAIDLARVLDLGPITRRGAQSPGVMNGTPSTAGF
LVAWPMVLLTVLLAWLF
 sein. Die genannten Peptide werden insbesondere für die Diagnose für die oben ausgeführten entzündlichen Darmerkrankungen beansprucht. Die genannten Sequenzen erfüllen im Wesentlichen dieselbe Funktion auf im Wesentlichen demselben Weg und bringen im Wesentliche dasselbe Ergebnis hervor wie die Sequenz SEQ ID Nr. 1. Sie werden daher von der erfindungsgemäßen Lehre, der Verwendung des Moleküls GP2 für die Verwendung als Arzneimittel zur Diagnose und/oder Therapiekontrolle von entzündlichen Darmerkrankungen erfasst.

Die Aminosäuresequenzen, d. h. die Peptide im Sinne der Erfindung, können daher soviel weitere Aminosäuren, Spacer oder andere Strukturen umfassen, dass sie geeignet sind Autoantikörpern zu interagieren, vorzugsweise so, dass sie ein Epitop für diese darstellen. Die erfindungsgemäße Sequenz ist demgemäß nicht auf die Peptide beschränkt, die Antikörper-Epitope betreffen, sondern sie bezieht sich auf das Molekül und alle Bruchstücke hiervon, die mit Autoantikörpern spezifisch so wechselwirken, dass eine Diagnose der entzündlichen Darmerkrankungen möglich ist. Die Begriffe Epitop und Peptid sowie Aminosäuresequenz können daher in bevorzugten Ausführungsformen im Sinne der Erfindung synonym verwendet werden.

Im Stand der Technik sind verschiedene Möglichkeiten zur Herstellung von funktionsanalogen Peptiden offenbart. Peptide, die von den erfindungsgemäßen Peptiden ausgehend mit solchen Verfahren designt werden, sind von der erfindungsgemäßen Lehre mit erfasst. Eine Möglichkeit des Generierens von funktionsanalogen Peptiden ist beispielsweise in PNAS USA 1998, Oct. 13; 9521:12179-84, WO 99/6293 und/oder WO 02/38592 beschrieben; diese Lehren sind in den Offenbarungsgehalten der Erfindung mit aufgenommen. Das heißt, sämtliche Peptide, Peptidfragmente oder Strukturen, die Peptide umfassen, die mit den genannten Verfahren - von den erfindungsgemäßen Peptiden ausgehend - generiert wurden, sind Peptide im Sinne der Erfindung, sofern sie die erfindungsgemäße Aufgabe lösen, insbesondere mit den krankheitsverursachenden Autoantikörpern wechselwirken. Bei diesen Autoantikörpern kann es sich beispielsweise um agonistische Autoantikörper handeln, die Rezeptoren aktivieren. In einer weiteren bevorzugten Ausführungsform der Erfindung erfolgt die Verwendung des Moleküls GP2 als lösliches oder festphasengebundenes Autoantigen zum direkten oder indirekten Autoantikörpernachweis in Stuhl und/oder Körperflüssigkeiten insbesondere Blut und/oder Serum, wobei sich hierbei als besonders vorteilhaft die Verwendung des Moleküls GP2 gemäß SEQ ID Nr. 1 erwiesen hat.

In einer weiteren bevorzugten Ausführungsform der Erfindung sind insbesondere die anmeldungsgemäßen Sequenzen bzw. Peptide, die aus diesen generiert werden können, immobilisiert. Insbesondere ist das festphasengebundene Molekül GP2 gemäß SEQ ID Nr. 1 an organische, anorganische, synthetische und/oder gemixte Polymere gebunden, vorzugsweise Agarose, Zellulose, Silica Gel, Polyamide und/oder Polyvinylalkohole. Im Sinne der Erfindung werden unter Immobilisierung auch verschiedene Verfahren und Techniken zum Fixieren der Peptide auf bestimmten Trägem beispielsweise gemäß der WO 99/56126 oder der WO 02/26292 verstanden. Die Immobilisierung kann beispielsweise der Stabilisierung der Peptide dienen, wodurch diese insbesondere bei Lagerung oder bei einmaligem Batch-Ansatz durch biologische, chemische oder physikalische Einwirkungen in ihrer Aktivität nicht reduziert oder nachteilig modifiziert werden. Durch die Immobilisierung der Peptide ist ein wiederholter Einsatz unter technischen oder klinischen Routine-Bedingungen möglich; weiterhin kann eine Probe - bevorzugt Blutbestandteile - mit mindestens einem der erfindungsgemäßen Peptide kontinuierlich umgesetzt werden. Dies kann insbesondere durch verschiedene Immobilisierungstechniken erreicht werden, wobei die Bindung der Peptide an andere Peptide oder Moleküle bzw. an einen Träger so erfolgt, dass die dreidimensionale Struktur, insbesondere an dem Zentrum, das die Wechselwirkung
mit den Autoantikörpern vermittelt, der entsprechenden Moleküle, insbesondere der Peptide, nicht verändert wird. Vorteilhafterweise geht die Spezifität zu den Autoantikörpern der Patienten durch die Immobilisierung nicht verloren. Im Sinne der Erfindung können drei grundsätzliche Methoden zur Immobilisierung verwendet werden:
(i) Quervernetzung: Bei der Quervernetzung werden die Peptide miteinander fixiert, ohne dass ihre Aktivität nachteilig beeinflusst wird. Sie sind vorteilhafterweise durch die Quervernetzung nicht mehr löslich.
(ii) Bindung an einen Träger: Die Bindung an einen Träger erfolgt zum Beispiel durch Adsorption, lonenbindung oder kovalente Bindung. Dies kann auch innerhalb von mikrobiellen Zellen bzw. Liposomen oder anderen membranhaltigen geschlossenen bzw. offenen Strukturen erfolgen. Die Peptide werden durch die Fixierung vorteilhafterweise nicht in ihrer Aktivität beeinflusst. Die Peptide können mit Vorteil zum Beispiel in der Klinik in Diagnose oder Therapie trägergebunden mehrfach oder kontinuierlich eingesetzt werden.
(iii) Einschluss: Der Einschluss erfolgt im Sinne der Erfindung insbesondere an eine semipermeable Membran in Form von Gelen, Fibrillen oder Fasern. Gekapselte Peptide sind durch eine semipermeable Membran so durch die umgebende Probenlösung getrennt, dass sie vorteilhafterweise noch mit den Autoantikörpern oder mit Fragmenten dieser interagieren können. Für die Immobilisierung stehen verschiedene Verfahren zur Verfügung, wie beispielsweise die Adsorption an einen inerten oder elektrisch geladenen anorganischen oder organischen Träger. Solche Träger können beispielsweise poröse Gele, Aluminiumoxid, Betonid, Agarose, Stärke, Nylon oder Polyacrylamid sein. Die Immobilisierung erfolgt hierbei durch physikalische Bindungskräfte, oft unter Beteiligung von hydrophoben Wechselwirkungen und ionischen Bindungen. Derartige Methoden sind vorteilhafterweise einfach zu handhaben und sie beeinflussen die Konformation der Peptide nur in geringem Umfang. Durch elektrostatische Bindungskräfte zwischen den geladenen Gruppen der Peptide und dem Träger kann die Bindung vorteilhafterweise verbessert werden, zum Beispiel durch die Verwendung von lonenaustauschern, insbesondere Sephadex.

Ein weiteres Verfahren ist die kovalente Bindung an Trägermaterialien. Die Träger können dazu reaktive Gruppen aufweisen, die mit Aminosäure-Seitenketten homöopolare Bindungen eingehen. Geeignete Gruppen in Peptiden sind Carboxy-, Hydroxy- und Sulfidgruppen und insbesondere die endständigen Aminogruppen von Lysinen. Aromatische Gruppen bieten die Möglichkeit für Diazo-Kopplungen. Die Oberfläche von mikroskopischen porösen Glaspartikeln kann durch Behandlung mit Silanen aktiviert und anschließend mit Peptiden umgesetzt werden. HydroxyGruppen natürlicher Polymere können zum Beispiel mit Bromzyan aktiviert und anschließend mit Peptiden gekoppelt werden. Mit Polyacrylamid-Harzen können zahlreiche Peptide vorteilhafterweise direkte kovalente Bindungen eingehen. Bei dem Einschluss in dreidimensionale Netzwerke werden die Peptide in ionotrophe Gele oder andere dem Fachmann bekannte Strukturen eingeschlossen. Die Poren der Matrix sind insbesondere so beschaffen, dass die Peptide zurückgehalten werden und eine Interaktion mit den Ziel-Molekülen möglich ist. Bei der Quervernetzung werden die Peptide durch Vernetzung mit bifunktionellen Agenzien in polymere Aggregate umgewandelt. Derartige Strukturen sind gelatinös und leicht verformbar und insbesondere für den Einsatz in verschiedenen Reaktoren geeignet. Durch Zugabe anderer inaktiver Komponenten, wie zum Beispiel Gelatine, bei der Vernetzung können die mechanischen und Bindungseigenschaften vorteilhafterweise verbessert werden. Bei der Mikroverkapselung wird der Reaktionsraum der Peptide mit Hilfe von Membranen eingegrenzt. Die Mikroverkapselung kann zum Beispiel als Grenzflächen-Polymerisation durchgeführt werden. Durch die Immobilisierung bei der Mikroverkapselung werden die Peptide unlöslich und dadurch wieder verwendbar. Im Sinne der Erfindung sind immobilisierte Peptide alle Peptide, die sich in einem Zustand befinden, der ihre Wiederverwendung erlaubt. Die Einschränkung der Beweglichkeit und der Löslichkeit der Peptide auf chemischem, biologischem oder physikalischem Wege führt vorteilhafterweise zu niedrigen Verfahrenskosten, insbesondere bei der Eliminierung von Autoantikörpern aus Blutbestandteilen.

In einer weiteren bevorzugten Ausführungsform der Erfindung werden zusätzlich zu dem löslichen oder festphasengebundenen Molekül GP2 gemäß SEQ ID Nr. 1 unspezifische Adsorbermoleküle verwendet, ausgewählt aus der Gruppe umfassend Protein A, Protein G, Anti-Human-Immunglobuline oder L-Tryptophan.

In einer weiteren bevorzugten Ausführungsform der Erfindung liegt das Molekül GP2 gemäß SEQ ID Nr. 1 linear oder in der zyklischen Form vor, wobei die Peptidzyklisierung bei Vorhandensein von zwei Zysteinen durch Disulfid-Brückenbindung erfolgt oder durch Amid-Zyklisierung, welche wahlweise. über die Seitenketten, über die terminalen C und N oder durch eine Kombination dieser Möglichkeiten erfolgt. Diese Variante des GP2 Moleküls kann bei den genannten Verfahren zu erhöhter Stabilität des GP2 Moleküls in Gegenwart verschiedener denaturierender Puffer führen.

In einem weiteren Aspekt betrifft die Erfindung ein immunogenes Mittel unter Verwendung von GP2 gemäß SEQ ID NO.1 zur Herstellung eines Medikaments zur Anwendung in der Diagnose oder Therapiekontrolle des oben genannten entzündlichen Darmerkrankungen, die mit einer Immunreaktion gegen diese Substanzen einhergehen. Überraschenderweise lassen sich mit den beschriebenen Sequenzen von GP2 auch einfach zu handhabende Medikamente herstellen, die der Patient nach Einweisung durch den Arzt selbst einnehmen oder verwenden kann. Auf diese Weise ist keine stationäre Behandlung notwendig. Die Beschreibung betrifft auch ein Diagnosetestbesteck (Kit) zur Bestimmung von Autoimmunerkrankungen, welches ein Molekül GP2 gemäß SEQ Nr. 1 umfasst. Gegebenenfalls enthält das Diagnosetestbesteck bzw. der Kit eine Anweisung zum Kombinieren der Inhalte des Testbestecks und/oder zur Bereitstellung einer Formulierung zur Detektion von entzündlichen Darmerkrankungen, umfassend Morbus Crohn, chronischer Pankreatitis und/oder Colitis ulcerosa. Diese Anweisung kann beispielsweise ein Beipackzettel sein oder ein anderes Medium, was dem Anwender Informationen darüber gibt, in welchem Verfahren die genannten Substanzen einzusetzen sind. Selbstverständlich ist es nicht zwingend erforderlich, dass die Information einen Beipackzettel darstellt, es ist möglich, dass diese Information beispielsweise über das Internet mitgeteilt wird. Dieser Kit hat für den Patienten beispielsweise den vorteilhaften Effekt, dass er sich ohne direkte Inanspruchnahme eines Arztes auch auf Reisen den aktuellen Grad seines Krankheitsstundes erfassen und gegebenenfalls seine Diät und seine Aktivitäten entsprechend anpassen kann.

Die anmeldungsgemäße Lehre zeichnet sich durch folgende Merkmale aus:
- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff)
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Diese Eigenschaften betreffen insbesondere die bevorzugten Ausführungsformen der Erfindung.

Im Folgenden soll die Erfindung anhand eines Beispiels näher erläutert werden, ohne auf dieses Beispiel beschränkt zu sein.

### Methoden

### Reinigung von GP2 aus Rattenpankreas

### Gewinnung des Zymogen-Granula (ZG) und Reinigung der ZG-Membranen

Alle folgenden Arbeitsschritte sind im Eisbad bzw. unter Kühlung auf 4°C Celsius durchgeführt worden. Das Pankreas von vier ausgewachsenen Wistar-Ratten (ca. 2,4 g Gewebe) wurde mechanisch zerkleinert und im zehnfachen Volumen von eiskalter 0,3 M Saccharose-Lösung im POTTER-Homogenisator aufgeschlossen (2 Hübe bei 1000 U/min und 2 Hübe bei 1300 U/min). Anschließend wurde der Aufschluss über ein Gaze-Tuch filtriert. Durch Zentrifugation bei 500 g für 10 min wurden Zelltrümmer und die Kerne abgetrennt. Aus dem Überstand wurden durch Zentrifugation bei 3000 g für 10 min die Zymogen-Granula (unteres, weißes festes Pellet) und die Mitochondrien (darüber-liegendes, lockeres bräunliches Pellet), abgeschieden. Die Mitochondrien sind mittels Puffer A (10 mM -Morpholinpropansulfonsäure (MOPS), pH 6,8) vorsichtig abgeschwämmt und die Zymogen-Granula in 2 ml 0,1 M Natrium-Karbonat Lösung, 1 mM Diisopropylfluorophosphat (DFP), mittels Vortexer resuspendiert worden. Die Lyse der Granula erfolgte 1 h im Eisbad. Der Lyse-Ansatz wurde über einen diskontinuierlichen Saccharose-Gradienten (0,3 M / 1 M) geschichtet und bei 200.000 g für 90 min zentrifugiert. Die Membranfraktion sammelte sich als Bande in der Dichte-Grenzschicht. Die Bande wurde abgesaugt und die gewonnene Lösung 0,3 M an Natrium-Bromid eingestellt. Durch Zentrifugation bei 200.000 g für 60 min wurden die Membranen sedimentiert.

### Solubilisierung des GP-2

Das gewonnene Membranpellet wurde in 0,5 ml Puffer B (20 mM Morpholinethansulfonsäure (MES), pH 7,0 ; 80 mM KCl; 45 µg/ml Saponin) mittels Ultraschall resuspendiert und nach Zusatz von Phosphatidylinosol-spezifischer Phospholipase C *(B. cereus )* durch Inkubation über 1 h bei 37°C GP2 von der Membran abgespalten. Die Membranen wurden durch Zentrifugation (200.000 g, 60 min) pelletiert und der Überstand mit dem löslichen GP-2 durch Ultrafiltration etwa 1 zu 5 konzentriert.

### Enzyme linked immosorbent assay (ELISA) zur Bestimmung von GP2 Antikörpern

Mikrotiterplatten (Maxisorb, Nunc, Roskilde) wurden mit 50µl/Kavität einer Lösung von 10µg/ml Ratten GP2 in Beschichtungspuffer (100 mM Na-Karbonat, pH 9,6), über Nacht bei 4°C beschichtet. Nach einmaligem Waschen der Mikrotiterplatte mit einem Waschpuffer (10 mM Na-Phosphat, 150 mM NaCl, 0,1% Tween20, pH 7,4) wurden die Kavitäten mit 300 µl der Blockierungslösung (Waschpuffer, 1 % Rinderserumalbumin (RSA), pH 7,4) für 30 min bei Raumtemperatur (RT) inkubiert. Anschließend wurden die Kavitäten dreimal mit Waschpuffer gewaschen und 50 µl 1/100 in Verdünnungspuffer (10 mM Na-Phosphat, 150 mM NaCl, 1% RSA) verdünnte humane Serumproben für 60 min bei RT pro Kavität inkubiert. Nach dreimaligem Waschen mit Verdünnungspuffer wurden die Kavitäten mit 50 µl Konjugatlösung (anti-human IgG-Peroxidase, Schaf, 1 µg/ml, Verdünnungspuffer) befüllt und für 30 min bei RT inkubiert. Anschließend wurden die Kavitäten erneut dreimal gewaschen und 50 µl der Substratfösung (Tetramethylbenzidin) pro Kavität dispensiert. Nach 10 min Inkubation bei RT erfolgte das Stoppen der Substratreaktion durch Zugabe von 50 µl Stopplösung (0,3 M Schwefelsäure). Die Optische Dichte der Lösung in den einzelnen Kavitäten wurde mittels Mikrotiterplattenphotometer bichromatisch bei 450 nm und 620 nm gemessen und Computer gestützt mit dem Softwareprogramm EIAstar ausgewertet.

### Indirekte lmmunfluoreszenz (IIF) zur Bestimmung von Pankreasantigen Antikörpern

Für die Bestimmung von Pankreasantigen Antikörpern mittel IIF wurden kommerzielle Affenpankreasschnitte (Euroimmun, Lübeck) verwendet. Die Serumproben wurden mit Verdünnungspuffer 1/40, 1/80 und 1/160 verdünnt. Je Reaktionsfeld des Reagenzträgers wurden 25 µl verdünntes Serum pipettiert und die Objektträger mit den Gewebeschnitten 30 min bei RT inkubiert. Anschließend wurden die Objektträger mit einem Phosphatpuffer für 1 min gewaschen. Je Feld des gereinigten Reagenzträgers sind dann 20 µl markiertes Antiserum (anti-human IgG FITC) pipettiert und mit den Gewebeschnitten auf den Objektträgern für 30 min bei RT inkubiert worden. Nach erneutem Waschen mit Phosphatpuffer für 1 min wurden die Objektträger mit Hilfe eines Eindeckmediums mit einem Deckglas versehen. Die Auswertung der Fluoreszenz erfolgte mit Hilfe eines Fluoreszenzmikroskops.

### Literatur

Bossuyt X. Serologic markers in inflammatory bowel disease. Clin Chem 2006, 52 (2): 171-181.

Fukuoka S-I. Molecular cloning and sequences of cDNAs encoding α (large) and β (small) isoforms of human pancreatic zymogen granule membraneassociated protein GP2. BBA 2000, 1491, 376-380

Fricke H, Birkhofer A, Folwaczny C, Meister W, Scriba PC. Characterization of antigens from the human exocrine pancreatic tissue (Pag) relevant as target antigens for autoantibodies in Crohn's disease. Eur J Clin Invest, 1999, 29: 41-45.

WO 01/94409 (CORIXA CORP [US]; Hirst Shannon K [US]; Harlocker Susan L [US]; Dillon) 13. Dezember 2001 (2001-12-13) Compositions and methods for the therapy and diagnosis of pancreatic cancer.

Main J, McKenzie H, Yeaman GR, Kerr MA, Robson D, Pennington CR, Parratt D. Antibody to saccharomyces cerevisiae (bakers' yeast) in Crohn's disease. BMJ, 297: 1105-1106.

Mayet WJ, Press AG, Hermann E, Moll R, Manns M, Ewe K, Meyer zum Büschenfelde KH. Antibodies to cytoskeletal proteins in patients with Crohn's disease. Eur J Clin Invest, 1990, 20: 516-524.

Seibold F, Weber P, Jenss H, Wiedmann K H. Antibodies to a trypsin sensitive pancreatic antigen in chronic inflammatory bowel disease: specific markers for a subgroup of patients with Crohn's disease. Gut 1991, 32: 1192-1197.

WO 96/17873 A (Alphagene INC [US]) 13. Juni 1996 (1996-06-13) Diagnosis of pancreatitits.

Stöcker W, Otte M, Ulrich S, Normann D, Finkbeiner H, Stöcker K, Jantschek G, Scriba PC. Autoimmunity to pancreatic juice in Crohn's disease. Results of an autoantibody screening in patients with chronic inflammatory bowel disease. Scand J Gastroenterol, 1987, 22 (suppl 139), 41-52.

## Patentansprüche

1. Verfahren zum Nachweis von Antikörpern aus Stuhl und/oder Körperfltissigkeiten insbesondere Blut und/oder Serum für die Diagnose und/oder Therapiekontrolle von entzündlichen Darmerkrankungen, ausgewählt aus der Gruppe umfassend Morbus Crohn, chronische Pankreatitis und/oder Colitis ulcerosa, durch eine lmmunreaktion mit GP2 gemäß SEQ ID Nr. 1, oder einem Molekül aufweisend eine Aminosäuresequenz, die eine Homologie zu SEQ ID Nr. 1 aufweist, wobei die Homologie mindestens 80%, vorzugsweise 90% beträgt, wobei die lmmunreaktion eine Bindung eines Antikörpers an ein Antigen umfasst und GP2 gemäß SEQ ID Nr. 1 das Antigen ist.

2. Verfahren nach Anspruch 1
**dadurch gekennzeichnet, dass**
der Nachweis in einem Immunoassay durchgeführt wird, vorzugsweise unter direkter oder indirekter Kopplung eines Reaktionspartners mit einer Markierungssubstanz unter Ausschluss von Immunfluoreszenztests auf der Basis von Gewebeschnitten.

3. Verwendung des Moleküls GP2 gemäß SEQ ID Nr. 1 als Antigen zur Diagnose und/oder Therapiekontrolle von entzündlichen Darmerkrankungen (EDE), wobei die Diagnose und/oder Therapiekontrolle ex vivo erfolgt.

4. Verwendung des Moleküls GP2 nach Anspruch 3,
**dadurch gekennzeichnet, dass**
die entzündliche Darmerkrankung Morbus Crohn, chronische Pankreatitis und/oder Colitis ulcerosa ist.

5. Verwendung des Moleküls GP2 nach einem der Ansprüche 3 oder 4,
**dadurch gekennzeichnet, dass**
das Molekül GP2 linear oder in der zyklischen Form vorliegt, wobei die Peptidzyklisierung bei Vorhandensein von zwei Zysteinen durch Disulfid-Brückenbindung erfolgt oder durch Amid-Zyklisierung, welche wahlweise über die Seitenketten, über die terminalen C und N oder durch eine Kombination dieser Möglichkeiten erfolgt.

6. Verwendung von GP2 gemäß SEQ ID Nr. 1 nach einem oder mehreren der vortierigen Ansprüche zur Diagnose und/oder Therapiekontrolle von entzündlichen Darmerkrankungen, wobei GP2 gemäß SEQ ID Nr. 1 löslich oder festphasengebunden zum direkten oder indirekten Autoantikörpemachweis in Körperflüssigkeiten, insbesondere Blut, Serum und/oder Stuhl verwendet wird.

7. Verwendung von GP2 gemäß SEQ ID Nr. 1 nach einem oder mehreren der vorherigen Ansprüche, wobei das festphasengebundene Molekül GP2 gemäß SEQ ID Nr. 1 an organische, anorganische, synthetische und/oder gemixte Polymere gebunden ist, vorzugsweise Agarose, Zellulose, Silica Gel, Polyamide und/oder Polyvinylalkohole.

8. Verwendung von GP2 gemäß SEQ ID Nr. 1 oder einem Molekül aufweisend eine Aminosäuresequenz, die eine Homologie zu SEQ ID Nr. 1 aufweist, wobei die Homologie mindestens 80%, vorzugsweise 90% beträgt, zur Herstellung eines Medikaments zur Anwendung in der Diagnose und/oder Therapiekontrolle von entzündlichen Darmerkrankungen, ausgewählt aus der Gruppe umfassend Morbus Crohn, chronische Pankreatitis und/oder Colitis ulcerosa, die mit einer Immunreaktion gegen diese Substanzen einhergehen, wobei GP2 gemäß SEQ ID Nr. 1 als Antigen eingesetzt wird und an Autoantikörper bindet.

9. Verwendung von GP2 bevorzugt gemäß SEQ ID Nr. 1 oder einem Molekül aufweisend eine Aminosäuresequenz, die eine Homologie zu SEQ ID Nr. 1 aufweist, wobei die Homologie mindestens 80%, vorzugsweise 90% beträgt, zur Herstellung eines Medikaments zur Anwendung in der oralen Diagnose und/oder Therapiekontrolle von entzündlichen Darmerkrankungen, ausgewählt aus der Gruppe umfassend Morbus Crohn, chronische Pankreatitis und/oder Colitis ulcerosa, die mit einer Immunreaktion gegen diese Substanzen einhergehen, wobei GP2 gemäß SEQ ID Nr. 1 als Antigen eingesetzt wird und an Autoantikörper bindet.

## Claims

1. Method for the detection of antibodies in stool and/or bodily fluids, especially blood and/or serum, for the diagnosis and/or treatment control of inflammatory intestinal diseases, selected from the group comprising Crohn's disease, chronic pancreatitis and/or ulcerative colitis, via an immune reaction with GP2 in accordance with SEQ ID No. 1, or a molecule with an amino acid sequence exhibiting a homology to SEQ ID No. 1, said homology being at least 80%, preferably 90%, whereby said immune reaction comprises the binding of an antibody to an antigen and GP2 in accordance with SEQ ID No. 1 is the antigen.

2. Method according to claim 1
**characterized in that**
the test is performed in an immunoassay, preferably under direct or indirect coupling of a coreactant with a marker substance under the exclusion of immunofluorescence tests on the basis of tissue sections.

3. Use of the molecule GP2 in accordance with SEQ ID No. 1 as antigen for the diagnosis and/or treatment control of inflammatory intestinal diseases (IID), said diagnosis and/or treatment control being performed ex vivo.

4. Use of the molecule GP2 according to claim 3,
**characterized in that**
the inflammatory intestinal disease is Crohn's disease, chronic pancreatitis and/or ulcerative colitis.

5. Use of the molecule GP2 according to one of the claims 3 or 4,
**characterized in that**
the molecule GP2 is present in linear or cyclic form, with peptide cyclization in the presence of two cysteines being effected by disulfide bonds or by amide cyclization, which is effected either via the side chains, via the terminal C and N or a combination of these possibilities.

6. Use of GP2 in accordance with SEQ ID No. 1 according to one or more of the preceding claims for the diagnosis and/or treatment control of inflammatory intestinal diseases, whereby GP2 in accordance with SEQ ID No. 1 in soluble or solid-phase bound form is used for direct or indirect antibody detection in bodily fluids, in particular blood, serum and/or stool.

7. Use of GP2 in accordance with SEQ ID No. 1 according to one or more of the preceding claims, whereby the solid-phase bound molecule GP2 in accordance with SEQ ID No. 1 is bound to organic, inorganic, synthetic and/or mixed polymers, preferably agarose, cellulose, silica gel, polyamides and/or polyvinyl alcohols.

8. Use of GP2 in accordance with SEQ ID No. 1 or a molecule with an amino acid sequence exhibiting a homology to SEQ ID No. 1, said homology being at least 80%, preferably 90%, for the manufacturing of a medicament to be applied in the diagnosis and/or treatment control of inflammatory intestinal diseases, selected from the group comprising Crohn's disease, chronic pancreatitis and/or ulcerative colitis, which involve an immune reaction against these substances, whereby GP2 in accordance with SEQ ID No. 1 is used as an antigen and binds to autoantibodies.

9. Use of GP2, preferably in accordance with SEQ ID No. 1, or a molecule with an amino acid sequence exhibiting a homology to SEQ ID No. 1, said homology being at least 80%, preferably 90%, for the manufacturing of a medicament to be applied in the oral diagnosis and/or treatment control of inflammatory intestinal diseases, selected from the group comprising Crohn's disease, chronic pancreatitis and/or ulcerative colitis, which involve an immune reaction against these substances, whereby GP2 in accordance with SEQ ID No. 1 is used as an antigen and binds to autoantibodies.

## Revendications

1. Procédé pour le dépistage d'anticorps dans les selles et/ou les fluides corporels tels que notamment le sang et/ou le sérum pour diagnostiquer et/ou suivre la thérapie de maladies intestinales inflammatoires de la catégorie regroupant la maladie de Crohn, la pancréatite chronique et/ou la colite ulcéreuse. Procédé se servant d'une réaction immunitaire avec GP2 selon SEQ ID n° 1 ou avec une molécule comprenant une séquence d'acide aminé ayant une homologie avec SEQ ID n° 1, cette homologie étant au moins de 80% et de préférence de 90% et la réaction immunitaire comprenant la liaison d'un anticorps avec un antigène et GP2 selon SEQ ID n° 1 étant cet antigène.

2. Procédé selon la revendication 1
**caractérisé en ce que**
le dépistage est réalisé dans le cadre d'un immunoessai, de préférence avec l'accouplement direct ou indirect entre un partenaire de réaction et une substance de marquage, le test d'immunofluorescence sur la base de coupes de tissus étant exclu.

3. Utilisation de la molécule GP2 selon SEQ ID n° 1 en tant qu'antigène pour diagnostiquer et/ou suivre la thérapie de maladies intestinales inflammatoires, le diagnostic et/ou le suivi de la thérapie étant réalisés ex vivo.

4. Utilisation de la molécule GP2 selon la revendication 3,
**caractérisée en ce que**
la maladie intestinale inflammatoire étant la maladie de Crohn, la pancréatite chronique et/ou la colite ulcéreuse.

5. Utilisation de la molécule GP2 selon l'une des revendications 3 ou 4,
**caractérisée en ce que**
la molécule GP2 est de forme linéaire ou cyclique, la cyclisation du peptide ayant lieu en cas de présence de deux cystéines par voie de pont disulfure ou par voie de cyclisation d'amides ayant lieu au choix via les chaînes latérales, via les extrémités C et N terminales ou par une combinaison de ces possibilités.

6. Utilisation de GP2 selon SEQ ID n° 1 selon l'une ou plusieurs des revendications susmentionnées pour le diagnostic et/ou le suivi de la thérapie de maladies intestinales inflammatoires, la molécule GP2 selon SEQ ID n° 1 soluble ou immobilisée sur phase solide étant utilisée pour le dépistage direct ou indirect d'autoanticorps dans les fluides corporels tels que notamment le sang, le sérum et/ou les selles.

7. Utilisation de GP2 selon SEQ ID n° 1 selon l'une ou plusieurs des revendications susmentionnées, la molécule GP2 selon SEQ ID n° 1 immobilisée sur phase solide étant liée avec des polymères organiques, inorganiques, synthétiques et/ou mixtes, de préférence l'agarose, la cellulose, le gel de silice, le polyamide et/ou les alcools de polyvinyle.

8. Utilisation de GP2 se!on SEQ ID n° 1 ou d'une molécule comprenant une séquence d'acide aminé ayant une homologie avec SEQ ID n° 1, cette homologie étant au moins de 80% et de préférence de 90%, pour produire un médicament prévu pour l'application dans le cadre du diagnostic et/ou du suivi de la thérapie de maladies intestinales inflammatoires de la catégorie regroupant la maladie de Crohn, la pancréatite chronique et/ou la colite ulcéreuse qui vont de paire avec une réaction immunitaire contre ces substances, GP2 selon SEQ ID n° 1 étant utilisée comme antigène et se reliant avec des autoanticorps.

9. Utilisation de GP2, de préférence selon SEQ ID n° 1, ou d'une molécule comprenant une séquence d'acide aminé ayant une homologie avec SEQ ID n° 1, cette homologie étant au moins de 80% et de préférence de 90%, pour produire un médicament prévu pour l'application dans le cadre du diagnostic oral et/ou du suivi de la thérapie de maladies intestinales inflammatoires de la catégorie regroupant la maladie de Crohn, la pancréatite chronique et/ou la colite ulcéreuse qui vont de paire avec une réaction immunitaire contre ces substances, GP2 selon SEQ ID n° 1 étant utilisée comme antigène et se reliant avec des autoanticorps.
